# EUROPÄISCHE PATENTSCHRIFT

(11) **EP 1 681 787 B1**
(45) Veröffentlichungstag und Bekanntmachung des Hinweises auf die Patenterteilung: **10.10.2007**
(21) Anmeldenummer: 06008583.4
(22) Anmeldetag: 14.09.2005
(51) Int. Cl.: H04B 10/22, G02B 6/36, A61B 6/00

(54) **Optischer Drehübertrager mit Reinigungsvorrichtung**
Optical rotary transmitter with a cleaning device
Transmetteur rotatif optique avec un dispositif de nettoyage

(30) Priorität: 24.09.2004 DE 102004046775; 24.09.2004 DE 102004046774; 24.09.2004 DE 102004046777; 02.12.2004 DE 102004058293; 02.12.2004 DE 102004058292; 02.12.2004 DE 102004058291
(43) Veröffentlichungstag der Anmeldung: 19.07.2006
(62) Teilanmeldung aus: 05020032.8
(73) Patentinhaber: Schleifring und Apparatebau GmbH, 82256 Fürstenfeldbruck (DE)
(72) Erfinder: Schilling, Harry, 85072 Eichstätt (DE); Tartler, Thomas, 82272 Moorenweis (DE); Thiele, Hans, 80689 München (DE); Lohr, Georg, 82223 Eichenau (DE); Hutterer, Rainer, 81247 München (DE); Rank, Matthias, 93497 Wilmering (DE)
(74) Vertreter: Lohr, Georg

(56) Entgegenhaltungen:
- DE-A1- 3 704 887
- DE-A1- 10 240 228
- DE-A1- 10 256 634

## Beschreibung

### Technisches Gebiet

Die Erfindung betrifft eine Vorrichtung zur Übertragung optischer Signale zwischen gegeneinander drehbaren Einheiten. Derartige Vorrichtungen werden vorzugsweise in Computertomografen eingesetzt.

### Stand der Technik

Zur Übertragung optischer Signale zwischen gegeneinander drehbaren Einheiten, insbesondere mit einem freiem Innendurchmesser sind verschiedene Vorrichtungen bekannt. Grundsätzlich besteht hierin das Problem, ein Mittel zum Transport von Licht entlang des Umfangs der Vorrichtung sowie geeignete Mittel zur ein- und Auskopplung von Licht zu gestalten. Zum Einsatz in Computertomografen müssen derartige Vorrichtungen große freie Innendurchmesser in einer Größenordnung von 1 Meter aufweisen. Die Umfangsgeschwindigkeit bei der Rotation kann in einer Größenordnung von 20 m/s liegen. Gleichzeitig sollten Datenraten mit über 1 Gigabit pro Sekunde (GBaud) möglich sein.

Nahezu alle Arten von optischen Drehübertragern reagieren empfindlich auf Verschmutzungen im Bereich des optischen Signalpfades. Auch in relativ sauberen Umgebungen, in denen beispielsweise Computertomografen eingesetzt werden, treten Staubpartikel auf. So wird beispielsweise in Computertomografen zur Energieversorgung der Röntgenröhre meist ein Schleifring mit einer Messingbahn und darauf laufenden Kohlebürsten eingesetzt. Diese Kohlebürsten werden im Betrieb durch das Schleifen auf der Messingbahn abgerieben und verursachen so einen extrem feinen Staub, welcher auch in feinste Öffnungen eindringen kann.

In der WO 03/069392 ist eine Vorrichtung zur breitbandigen Signalübertragung mittels eines in Längsrichtung geteilten Lichtleiters offenbart. Es ist der Inhalt der WO 03/069392 durch Bezugnahme mit in dieses Dokument aufgenommen. Der Lichtleiter liegt hier frei und damit ungeschützt vor Staub und anderen Verschmutzungen.

### Darstellung der Erfindung

Der Erfindung liegt die Aufgabe zugrunde, eine Vorrichtung zur Übertragung optischer Signale zwischen zwei gegeneinander drehbaren Einheiten, vorzugsweise zum Einsatz in Computertomographen, derart zu gestalten, dass Staub und anderen Verschmutzungen, insbesondere von Schleifbahnen, die Übertragungsqualität nicht beeinträchtigen können.

Erfindungsgemäße Lösungen dieser Aufgabe sind in den unabhängigen Patentansprüchen angegeben. Weiterbildungen der Erfindung sind Gegenstand der abhängigen Ansprüche.

Eine erfindungsgemäße Vorrichtung umfasst einen optischen Drehübertrager mit Lichtleiter 3, welcher entlang einer Kreisbahn an einer ersten Einheit 1 angeordnet ist. Der Einfachheit halber wird hier nur ein Lichtleiter beschrieben. Selbstverständlich können auch mehrere Lichtleiter in einer erfindungsgemäßen Vorrichtung vorgesehen werden. Zum Abgriff der Signale aus dem Lichtleiter 3 ist an einer zweiten Einheit 2, welche gegenüber der ersten Einheit 1 drehbar gelagert ist, ein Lichtkoppler 5 vorgesehen. Weiterhin ist eine Reinigungseinheit 40,41 zur Entfernung von Schmutz und/oder Staubpartikeln von dem Lichtleiter 3 vorgesehen. Die Aufgabe der Reinigungseinheit ist es primär, Verschmutzungen von dem Lichtleiter 3 zu entfernen. Weiterhin können selbstverständlich auch Verschmutzungen von umgebenden Bauteilen, wie einer Lagerung, insbesondere einer Luftlagerung entfernt werden.

In einer weiteren Ausgestaltung der Erfindung ist wenigstens eine Reinigungseinheit 40 mit wenigstens einer zweiten Einheit 2 derart verbunden, so dass diese zusammen mit der zweiten Einheit 2 entlang des Lichtleiters 3 bewegbar ist. Somit bewegt sich die Reinigungseinheit zusammen mit der Bewegung der zweiten Einheit. Dadurch lässt sich eine einfache Halte- und Antriebs- Konstruktion für die Reinigungseinheit 40 realisieren. Besonders günstig ist es, wenn die Reinigungseinheit in Bewegungsrichtung vor einem Lichtkoppler 5 geführt wird, so dass der Lichtleiter vor der Übertragung gereinigt wird. Bei Ausführungen mit flüssigen Reinigungsmitteln kann es dagegen besonders vorteilhaft sein, die Reinigungseinheit in Bewegungsrichtung hinter einem Lichtkoppler 5 zu führen, da dann eine eventuell auf dem Lichtleiter verbliebene Reinigungsflüssigkeit trocknen kann.

Eine weitere Ausgestaltung der Erfindung sieht eine Reinigungseinheit 41 vor, welche unabhängig von einer zweiten Einheit entlang des Lichtleiters 3 bewegbar ist. Mit dieser Ausgestaltung kann eine unabhängige Steuerung der Bewegung der Reinigungseinheit 41 von der Bewegung eines Lichtkopplers 5 erfolgen. So könnten beide mit unterschiedlichen Geschwindigkeiten bewegt werden, um beispielsweise eine Anpassung der Geschwindigkeit an den Grad der Verschmutzung zu erreichen. Entsprechend können auch mehrere Reinigungseinrichtungen vorgesehen, welche sich in festen Abständen oder auch unabhängig voneinander entlang des Lichtleiters bewegen können. Entsprechend der Ausgestaltung der Reinigungseinheit 41 kann die Bewegung auf der gleichen Bahn wie die eines Lichtkoppler 5 erfolgen. Dabei ist es unmöglich, dass eine Reinigungseinheit 41 einen Lichtkoppler 5 überholt. Dennoch kann die lokale Geschwindigkeit der Reinigungseinheit 41 angepasst werden. So könnte diese beispielsweise bei einer besonders stark verschmutzten Stelle reduziert und nach Reinigung dieser Stelle zumindest kurzzeitig erhöht werden, um eine Kollision mit einem Lichtkoppler 5 zu vermeiden. Alternativ kann eine Reinigungseinheit 41 auch derart ausgestaltet werden, dass sie eine andere Bahn als ein Lichtkoppler 5 verwendet. Hier kann eine solche Reinigungseinheit 41 vollständig unabhängig von einem Lichtkoppler 5 bewegt werden. Dazu ist dann die Reinigungseinheit 41 vorzugsweise seitlich oder oberhalb der Bahn eines Lichtkopplers 5 anzuordnen.

Eine andere Ausgestaltung der Erfindung sieht einen Sensor zur Erkennung von Verschmutzung vor. Weiterhin ist eine optionale Steuereinheit vorgesehen, welche abhängig von Verschmutzungsgrad die Einwirkzeit und/oder Reinigungsintensität die Reinigungseinheit einstellt. So kann beispielsweise bei besonders starker Verschmutzung mit höherem Druck, höherer Temperatur, oder höherer Flüssigkeitsmenge gereinigt werden, während beispielsweise bei sehr geringer oder nicht nachweisbarer Verschmutzung die Reinigungseinrichtung abgeschaltet wird. Dadurch lässt sich ein niedrigerer Energieverbrauch sowie gegebenenfalls ein geringerer Verbrauch an Reinigungsmitteln erreichen.

Eine vorteilhafte Ausgestaltung der Erfindung sieht eine Einrichtung zum Ausblasen von Staub und Schmutzpartikeln vor. Hierzu wird vorzugsweise mit einer Düse 42 Luft in einem Luftstrom 44 auf den Lichtleiter 3 geblasen. Neben Luft sind selbstverständlich auch andere Gase oder Flüssigkeiten geeignet. Zur Speisung der Düse mit einem Luftstrom 43 ist eine Luftdruckquelle bzw. Luftpumpe vorgesehen. Durch den Luftstrom werden die Staub bzw. Schmutzpartikel von der Oberfläche des Lichtleiters 3 aufgewirbelt und fortgerissen. Besonders günstig ist es, die Düse derart anzuordnen, dass die aufgewirbelten Partikel seitlich von dem Lichtleiter weggeblasen werden. Zur Unterstützung dieses Effekts können auch Luftleitvorrichtungen, wie Luftleitbleche vorgesehen sein. Vorteilhafterweise weist die Luftpumpe ein Filter auf, so dass ausschließlich gereinigte Luft auf den Lichtleiter geblasen wird. Eine weitere Verbesserung der Reinigungswirkung lässt sich durch die Erzeugung von Wirbeln, welche auf der Oberfläche des Lichtleiters eine lokal begrenzte, hohe Strömungsgeschwindigkeit besitzen, erreichen. Hierbei kann es solche vorteilhaft sein, den Luftstrom 44 entgegen der Bewegungsrichtung der Reinigungseinheit zu richten.

Bei Anwendungen, in denen ein Luftlager zu Abstützung eines Lichtkopplers 5 gegenüber dem Lichtleiter 3 vorgesehen ist, kann die Strömung zum Wegblasen der Staub- und Schmutzpartikel durch zusätzliche Strömungskanäle zur Führung der ohnehin für die Lagerung benötigten Luft erzeugt werden.

In einer weiteren vorteilhaften Ausgestaltung der Erfindung weist eine Reinigungseinheit 40, 41 eine Saugvorrichtung auf. Eine solche Saugvorrichtung wird an eine Unterdruckquelle, wie beispielsweise eine Luftpumpe angeschlossen. Sie weist eine Absaugöffnung 60 auf, durch welche die Luft von der Oberfläche des Lichtleiters 3 abgesaugt wird. Diese Absaugöffnung ist vorzugsweise so angeordnet, dass sie zumindest in einem engen Bereich an der Oberfläche des Lichtleiters eine möglichst hohe Strömungsgeschwindigkeit erzeugt. Diese Strömungsgeschwindigkeit sollte so hoch bemessen sein, dass ein hoher Anteil an Schmutzpartikeln mitgerissen wird. Vorteilhafterweise ist in dem Luftstrom noch ein Filter vorgesehen, welches die Staub und Schmutzpartikel zurückhält, so dass diese nicht weiter an die Umgebungsluft abgegeben werden. Eine solche Saugvorrichtung lässt sich sehr vorteilhaft mit der oben beschriebenen Einrichtung zum Ausblasen kombinieren. In diesem Fall kann es ausreichend sein, wenn die Einrichtung zum Ausblasen eine hohe Strömungsgeschwindigkeit der Luft erzeugt. Dieser Vorrichtung muss dann nur noch die aufgewirbelten Partikel entfernen, benötige hierzu aber keine hohe Strömungsgeschwindigkeit.

Eine weitere vorteilhafte Ausgestaltung der Erfindung sieht eine Bürsteneinheit zur Reinigung der Oberfläche des Lichtleiters vor. Diese Bürsteneinheit umfasst typischerweise einen Bürstenträger 52, welcher eine oder mehrere Bürsten 53 aufnimmt. Die Bürsten sind so angeordnet, dass sie entlang der Oberfläche des Lichtleiters 3 gleiten. Die Bürsten können wie Pinsel, aber auch in Form einer Walze ausgebildet sein. Vorteilhafterweise sind mehrere Bürsten in Bewegungsrichtung hintereinander angeordnet, um eine besonders gründliche Reinigung zu erreichen. Weiterhin können Bürsten unterschiedlicher Härtegrade miteinander kombiniert werden, um verschiedene Partikelgrüßen von unterschiedlichen Verschmutzungen von dem Lichtleiter 3 zu entfernen Besonders vorteilhaft ist auch hier die Kombination einer Bürsteneinheit mit der zuvor beschriebene Saugvorrichtung und/oder der zuvor beschriebenen Einrichtung zum Ausblasen. Dadurch kann eine wesentlich stärkere Reinigungswirkung, als mit nur einer dieser Vorrichtungen alleine erreicht werden.

In einem erfindungsgemäßen Verfahren erfolgt die Reinigung der Oberfläche eines Lichtleiters 3 in der oben angegebenen Anordnung zu Übertragung optischer Signale durch Erzeugen eines Luftstroms 44 mit einer Düse 42 und Ausblasen von Staub und Schmutzpartikeln von der Oberfläche des Lichtleiters 3.

Ein weiteres erfindungsgemäßes Verfahren zur Reinigung der Oberfläche eines Lichtleiters 3 umfasst das Absaugen von Staubpartikeln und Schmutzpartikeln von der Oberfläche des Lichtleiters 3.

Ein weiteres erfindungsgemäßes Verfahren zur Reinigung der Oberfläche eines Lichtleiters 3 umfasst das Abbürsten von Staubpartikeln und Schmutzpartikeln von der Oberfläche des Lichtleiters 3.

Ein weiteres erfindungsgemäßes Verfahren zur Reinigung der Oberfläche eines Lichtleiters 3 umfasst die Abgabe eines Dampfstroms 56, welcher auf den Lichtleiter 3 gerichtet ist.

Ein weiteres erfindungsgemäße Verfahren zur Reinigung der Oberfläche eines Lichtleiters 3 umfasst die Abgabe einer Reinigungsflüssigkeit 48 auf den Lichtleiter 3 sowie die Aufnahme der Reinigungsflüssigkeit 48 mit darin gelösten Schmutzpartikeln 39.

Eine weitere erfindungsgemäße Vorrichtung umfasst einen optischen Drehübertrager mit Lichtleiter 3, welcher entlang einer Kreisbahn an einer ersten Einheit 1 angeordnet ist. Der Einfachheit halber wird hier nur ein Lichtleiter beschrieben. Selbstverständlich können auch mehrere Lichtleiter in einer erfindungsgemäßen Vorrichtung vorgesehen werden. Zum Abgriff der Signale aus dem Lichtleiter 3 ist an einer zweiten Einheit 2, welche gegenüber der ersten Einheit 1 drehbar gelagert ist, ein Lichtkoppler 5 vorgesehen. Die erste Einheit 1 und die zweite Einheit 2 sind derart ausgebildet, dass sie gemeinsam wenigstens einen Lichtleiter 3 umschließen. In dem umschlossenen Raum mit dem Lichtleiter 3 ergibt sich ein Innenbereich 35. Außerhalb der gesamten Anordnung besteht der Außenbereich 36. Zwischen Innenbereich und Außenbereich verlaufen Spalte 37, 38, welche durch die drehbare Anordnung der beiden Einheiten gegeneinander bedingt sind. Weiterhin ist eine Vorrichtung zur Separation des Innenbereichs gegenüber dem Außenbereich vorgesehen. Diese Vorrichtung ist derart ausgelegt, dass ein Eindringen von Schmutz und /oder Staubpartikeln aus dem Außenbereich in den Innenbereich erschwert oder verhindert wird. Sie arbeitet bevorzugterweise auf hydrodynamische Art.

In einer besonders vorteilhaften Ausgestaltung der Erfindung wird diese hydrodynamische Vorrichtung aus Luft versorgt, welche bereits zum Betrieb einer hydrostatischen oder hydrodynamischen Lagerung, insbesondere einer Luftlagerung verwendet wurde.

In einer weiteren vorteilhaften Ausgestaltung der Erfindung wird durch wenigstens eine Sperrluftdüse 51, 52 zusätzliche Sperrluft 55 in die Spalte 37 bzw. 38 geblasen. Diese Sperrluft sorgt zumindest für einen von dem Innenbereich in den Außenbereich verlaufenden Luftstrom, welcher ein Eindringen von störenden Partikeln erschwert. Für eine optimale Wirkung sollte die Höhe 57 eines Spalts 37 in einem Bereich von 0,03 bis 0,1 mm sein. Besonders vorteilhaft ist eine Höhe in einer Größenordnung von 0,6 mm. Ein Druck der Sperrluft 55 am Austrittsort durch die Sperrluftdüse von 0,2 bis 0,5 bar hat sich besonders bewährt. Zum Austritt des nach innen gehenden Anteils der Sperrluft sowie der optionalen Zuluft 54 ist vorteilhafterweise ein Abluftventil 53 vorgesehen. Zu einer verbesserten Verteilung der Sperrluft entlang des Umfangs der Anordnung ist es vorteilhaft, zusätzliche ringförmige Nuten im Bereich wenigstens eines Spalts 37 bzw. 38 vorzusehen. Ebenso ist es vorteilhaft, die Sperrluft an mehreren Stellen, vorzugsweise entlang des Kreisumfangs einzuspeisen. Besonders vorteilhaft ist es, beide Spalte 37 bzw. 38 durch Sperrluft gegenüber dem Eindringen von Schmutz und Staub abzudichten.

Eine andere vorteilhafte Ausgestaltung der Erfindung sieht vor, dass wenigstens eine Labyrinthdichtung 58a, 58b im Bereich der Spalte 37 bzw. 38 vorgesehen ist. Zur Ausbildung der Labyrinthdichtung weisen die erste Einheit sowie die zweite Einheit im Bereich der Spalte Vertiefungen, insbesondere ringförmige Nuten, sowie darin eingreifende Erhöhungen, insbesondere Stege auf der Gegenseite auf. Wesentlich ist, dass die Erhöhungen sowie die Vertiefungen ineinander greifen. Dadurch wird die Weglänge zwischen dem Außenbereich und den Innenbereich vergrößert .Weiterhin erhöht sich der Strömungswiderstand. Durch eine scharfkantige Ausbildung entstehen zusätzliche Wirbel, welche zu einer Ablagerung von Verschmutzungen im Labyrinth führen, vor diese in den Innenraum eindringen. Durch die Erhöhung des Strömungswiderstandes genügt eine geringere Luftmenge zur Speisung des Innenbereichs mit Sperrluft.

Eine weitere Ausgestaltung der Erfindung sieht Bürsten 60 zwischen dem Innenbereich und dem Außenbereich im Bereich der Spalte vor. Hierdurch kann eine relativ gute Dichtwirkung insbesondere in Verbindung mit engen Spalten bzw. Labyrinthen erreicht werden. Diese Ausgestaltung erlaubt auch einen Schutz des Innenraumes im Stillstand und ohne Luftversorgung. In den Bürsten angesammelter Staub und Schmutz kann durch einen höheren Luftdruck nach außen ausgeblasen werden.

In einer anderen Ausgestaltung der Erfindung sind Filzdichtungen 61a, 61b zwischen dem Innenbereich und dem Außenbereich im Bereich der Spalte vorgesehen. Auch hierdurch ergibt sich eine besonders gute Dichtwirkung, welche auch im Stillstand und ohne Luftversorgung aus dem Innenbereich anhält. Durch einen erhöhten Luftdruck von der Innenseite kann dafür gesorgt werden, dass ein eventuell notwendiges Schmiermittel nicht in den Innenbereich eindringen kann.

Eine weitere Ausgestaltung der Erfindung sieht den Einsatz von Gleitringdichtungen vor. Wenigstens ein Gleitring 62 mit passendem Gegenring 63 ermöglicht eine gute Dichtung zwischen Innenbereich und Außenbereich. Vorteilhafterweise werden zwei Gleitringdichtungen miteinander kombiniert, so dass sie einen abgeschlossenen Zwischenraum zwischen dem Innenbereich und dem Außenbereich ausbilden. Dieser Zwischenraum kann dann vorteilhafterweise mit einem Sperrmedium oder mit einem Schmiermittel bzw. Kühlmedium für die Gleitringdichtungen gefüllt werden. Dieses kann durch Öffnungen 66 zugeführt und durch Öffnungen 67 abgeführt werden. Da durch die Gleitringdichtungen ein Luftaustritt aus dem Innenraum nicht mehr möglich ist, ist ein Abluftventil 53 zur Abführung der Zuluft 54 notwendig. Eine solche Ausgestaltung mit Gleitringdichtungen ist weitgehend wartungsfrei. Entstehender Abrieb kann durch das Schmiermittel aufgenommen und abgeführt werden. Der Innenbereich ist auch bei Stillstand und ohne Luftversorgung vor Verunreinigung geschützt.

Eine andere Ausgestaltung der Erfindung sieht den Einsatz von wenigstens einer Radialwellendichtung vor. Wenigstens eine solche Radialwellendichtung 64 ist in einem Spalt zwischen Innenbereich und Außenbereich vorgesehen. Vorteilhafterweise sind zwei solcher Dichtungen hintereinander vorgesehen, so dass diese einen abgeschlossenen Zwischenraum ausbilden, welcher wie bereits zuvor dargestellt, mit einem Sperrmedium, einem Schmiermittel oder Kühlmittel gefüllt bzw. versorgt werden kann. Auch hier kann ein eventuell entstehende Abrieb durch dieses Mittel abgeführt werden. Ebenfalls ist hier der Innenbereich bei Stillstand und ohne Luftversorgung vor Verunreinigung geschützt.

In einer weiteren vorteilhaften Ausgestaltung der Erfindung ist eine lichtdurchlässige Abdeckung 71 vorgesehen, welche mit der ersten Einheit 1 fest verbunden ist. Diese lichtdurchlässige Abdeckung dichtet zusammen mit der ersten Einheit den Lichtleiter 3 gegenüber dem Außenbereich ab. Weiterhin ist zur optischen Verkopplung mit dem Lichtleiter 3 wenigstens ein optisches Element zur freien Strahlführung durch die lichtdurchlässige Abdeckung vorgesehen. Ebenso wie der ungeschützte Lichtleiter kann auch die lichtdurchlässige Abdeckung verschmutzen. Der Vorteil der lichtdurchlässigen Abdeckung liegt aber darin, dass diese beispielsweise als plane Fläche und damit leicht reinigbar ausgebildet werden kann. Weiterhin ist es vorteilhaft, wenn der freie durch die lichtdurchlässige Abdeckung hindurchtretende Lichtstrahl gegenüber dem im Lichtleiter 3 geführten Strahl aufgeweitet wird. Damit ist auch noch bei einzelnen auf der Oberfläche liegenden Staub bzw. Schmutzpartikeln eine Übertragung möglich. Weiterhin kann die Optik zur Übertragung durch die lichtdurchlässige Abdeckung so ausgebildet sein, dass die lichtdurchlässige Abdeckung außerhalb des Fokus, vorzugsweise in einem parallelen Lichtstrahl liegt.

In einer weiteren Ausgestaltung der Erfindung ist ein Staubfilter 73, 74 zwischen der ersten Einheit und der zweiten Einheit im Bereich wenigstens eines Spalts vorgesehen. Das Filter schließt den Spalt zumindest für Staub und Schmutzpartikel dicht ab, lässt aber Luft von der Innenseite entweichen. Auch diese Ausbildung ist im Stillstand und ohne Luftversorgung dicht in Bezug auf Staub und Schmutzpartikel. Ein vollgesetztes Filter lässt sich leicht durch erhöhten Luftdruck von der Innenseite aus reinigen. Das Filter besteht vorzugsweise aus einem Gewebe, wie einer Gaze oder auch einem Filterpapier. Ein optimales zusätzliches Filter 75 weist ein größeres Fassungsvolumen auf und kann auch gröbere Verunreinigungen ausfiltern.

In einer weiteren vorteilhaften Ausgestaltung der Erfindung ist noch ein zusätzliches Filter 75 als elektrostatisches Filter ausgebildet.

Eine andere Ausgestaltung der Erfindung sieht eine Dichtmembran 80 an einer der beiden Einheiten vor, welche vor einem Hohlraum 81 angebracht ist und durch Beaufschlagung des Hohlraumes 81 mit Vakuum bzw. Druckluft 82 bewegt werden kann. Durch Beaufschlagung mit Druckluft kann die Dichtmembran an eine diese gegenüberliegende Fläche, welche vorzugsweise der anderen Einheit zugeordnet ist angenähert beziehungsweise angedrückt werden. Durch Beaufschlagung mit Vakuum wird die Luft zumindest teilweise aus dem Hohlraum gesaugt und die Membran in diesen hineingezogen, sodass sich der Abstand zur gegenüberliegende Fläche vergrößert und damit die Dichtung geöffnet wird. Vorteilhafterweise wird die Membran so dimensioniert, dass sie im Ruhezustand leicht an der gegenüberliegenden Fläche anliegt, um eine gewisse Mindestdichtung in diesem Ruhezustand zu erreichen. Weiterhin wird Vorteilhafterweise an der gegenüberliegenden Fläche eine Dichtung angebracht, um eine definierte Auflagefläche zu Dichtmembran zu herzustellen.

In einer weiteren erfindungsgemäßen Anordnung ist wenigstens ein Gleitkörper 2a vorgesehen, welcher zur Aufnahme eines zweiten Lichtkopplers 5 dient. Weiterhin ist dieser Gleitkörper mit einer magnetischen Lagerung versehen. Die magnetische Lagerung dient zur präzisen Führung entlang des Lichtleiters 3. Sie kann sowohl statisch als auch dynamisch ausgebildet sein. So können wahlweise Permanentmagnete als auch Elektromagnete zur Erzeugung der Magnetfelder vorgesehen sein. Weiterhin ist es vorteilhaft, eine zusätzliche, vorzugsweise elektronisch gesteuerte Lageregelung vorzusehen.

In einem erfindungsgemäßen Verfahren zur Abdichtung einer optischen Übertragungseinheit entsprechend dem Oberbegriff des Anspruchs 1 gegenüber Schmutz und Staub wird diese in ihrem Innenbereich 35 mit einem erhöhten Luftdruck gegenüber dem Außenbereich 36 beaufschlagt, so dass Luft durch Öffnungen in der Oberfläche, wie Spalte nach außen strömt und das eindringen von Partikeln verhindert.

Aus Gründen der übersichtlicheren Darstellung wurde in den vorhergehenden Ausführungen auf den Begriff Luft stellvertretend für beliebige Gase Bezug genommen. Selbstverständlich ist eine erfindungsgemäße Vorrichtung bzw. eine erfindungsgemäßes Verfahren mit beliebigen anderen Gasen, vorzugsweise mit Stickstoff realisierbar.

Eine weitere erfindungsgemäße Vorrichtung umfasst einen optischen Drehübertrager mit Lichtleiter 3, welcher entlang einer Kreisbahn an einer ersten Einheit 1 angeordnet ist. Der Einfachheit halber wird hier nur ein Lichtleiter beschrieben. Selbstverständlich können auch mehrere Lichtleiter in einer erfindungsgemäßen Vorrichtung vorgesehen werden. Zum Abgriff der Signale aus dem Lichtleiter 3 ist an einer zweiten Einheit 2, welche gegenüber der ersten Einheit 1 drehbar gelagert ist, ein Lichtkoppler 5 vorgesehen.

Untersuchungen haben ergeben, dass die bei schleifenden Kontaktanordnungen entstehenden Abriebpartikel von elektrisch geladenen Gegenständen angezogen werden. Dies ist eine Folge der elektrostatischen Aufladung während des Schleifens. Zudem erhalten die Partikel durch das Potenzial, mit dem die Kontaktanordnung beaufschlagt ist, eine zusätzliche Ladung.

Daher wird erfindungsgemäß eine Vorrichtung zur elektrostatischen Filterung und/oder Beseitigung der Abriebpartikel vorgesehen. Eine solche Vorrichtung ist zudem in der Lage, auch fremde Staub- und Schmutzpartikel zu entfernen. Ebenso kann eine erfindungsgemäße elektrostatische Vorrichtung dazu eingesetzt werden, den Staub von der Schleifkontaktvorrichtung zu entfernen, um eine Beeinträchtigung der Isolation zu vermeiden.

In einer besonders vorteilhaften Ausgestaltung der Erfindung ist die elektrostatische Vorrichtung als elektrostatisches Luftfilter ausgebildet. Dieses weist wenigstens eine mit Hochspannung beaufschlagbare Elektrode auf. Da sehr viele der Abriebpartikel bereits an die Umgebungsluft abgegeben werden und dort als feiner Staub verteilt sind, werden mit der erfindungsgemäßen elektrostatischen Filteranordnung diese in der Luft befindlichen und geladenen Partikel herausgefiltert.

Weiterhin ist vorteilhafterweise wenigstens eine Coronaelektrode vorgesehen, welche mit einer Hochspannung beaufschlagbar ist und eine Aufladung der noch nicht aufgeladenen Partikel in der Luft vornimmt. Dies kann auch über eine Ionisierung der Luft erfolgen. Die Coronaelektrode ist vorzugsweise als dünner Draht ausgebildet, um eine möglichst hohe elektrische Feldstärke in der Nähe der Elektrode zu erreichen.

Eine weitere Ausgestaltung der Erfindung sieht vor, dass wenigstens ein Mittel zur Erzeugung eines Luftstroms vorhanden ist. Ein solches Mittel kann aktiver Art sein, wie beispielsweise ein Gebläse oder ein Ventilator. Ebenso kann dieses Mittel auch ein Mittel zur Luftführung, wie Luftleitbleche oder Luftkanäle sein. Selbstverständlich sind beide Arten der Mittel kombinierbar. In vielen Anwendungsfällen sind einfache Mittel zur Luftführung, wie Luftkanäle ausreichend, da bereits durch die Bewegung der zweiten Einheit gegenüber der ersten Einheit ein hinreichender Luftstrom entsteht. Es muss dieser lediglich noch in die richtige Richtung gesteuert werden, sodass er bevorzugt zuerst die Coronaelektrode passiert und dann an der zweiten flächigen Elektrode vorbei geleitet wird. Besonders günstig ist es, eine solche Filteranordnung mit den zugehörigen Mitteln zur Steuerung der Strömung vor den optischen Komponenten anzuordnen, sodass diese bereits eine gereinigte Luft erhalten.

Es kann erfindungsgemäß mindestens ein Gebläse vorgesehen sein, welches einen Luftstrom über der Kontaktanordnung oder über dem Lichtleiter 3 oder einem Lichtkoppler 5 erzeugt. Damit kann das Entfernen des Abriebs weg von der Kontaktzone und deren Umfeld zusätzlich sichergestellt und die Entfernungswirkung mittels der Elektrode unterstützt und/oder verstärkt werden. Der Abrieb wird dabei durch den Luftstrom gerichtet entfernt. Es können auch Luftkanäle oder Luftleitbleche vorgesehen sein.

Besonders günstig ist es, Mittel zur elektrostatischen Luftfilterung mit anderen, vorzugsweise mechanischen Filtern, beispielsweise Maschenfiltern oder Papierfiltern zu kombinieren, um eine besonders hohe Filterwirkung zu erreichen.

Eine weitere erfindungsgemäße Ausgestaltung sieht vor, dass wenigstens ein Mittel zur Aufnahme von Staub- und Schmutzpartikeln nahe der Oberfläche einer der beiden Einheiten angeordnet ist. Dieses wird mit Hochspannung beaufschlagt, so dass geladene Staub- und Schmutzpartikeln angezogen werden. Somit wird nicht primär die das System durchströmende Luft gereinigt, vielmehr werden geladene Partikel von einer Oberfläche abgezogen.

Weiterhin vorteilhaft ist es, wenn dieses Mittel als drehbarer Körper, vorzugsweise als Rolle ausgebildet ist. Damit kann während der Bewegung über die Oberfläche der ersten oder der zweiten Einheit immer ein sauberer Bereich des drehbaren Körpers in den Nähe der ersten oder zweiten Einheit gebracht werden. Gleichzeitig kann in einem kontinuierlichen Prozess die Oberfläche des drehbaren Körpers gereinigt werden. Ebenso kann einem kontinuierlichen Prozess die Oberfläche des drehbaren Körpers wieder mit elektrischen Ladungen aufgeladen werden.

In einer weiteren vorteilhaften Ausgestaltung der Erfindung, besteht zumindest eine der Elektroden aus einem Material, das eine Oberfläche mit hoher Adhäsion für die Abriebpartikel besitzt. Dadurch werden einmal angezogene Abriebpartikel dauerhaft, auch bei Ausfall der Elektrodenspannung, an die Elektrode gebunden. Derartige Materialien können beispielsweise gummiartige Materialien sein, an denen Kohlestaub gut haftet oder andere Materialien, wie sie als Klebebänder verwendet werden.

In einer weiteren vorteilhaften Ausgestaltung der Erfindung, wird zumindest ein zusätzliches Gebläse vorgesehen, um die verunreinigte Luft von der Kontaktstelle zu den Elektroden weiterzuleiten. Der Abrieb wird dabei durch den Luftstrom gerichtet zu den Elektroden entfernt.

In einer weiteren Ausgestaltung der Erfindung ist wenigstens eine Elektrode als austauschbare Einweg-Elektrode ausgebildet.

Alternativ hierzu kann somit ist ein Teil der Filteranordnung als austauschbare Einweg-Baugruppe ausgebildet sein. Durch Einweg-Elektroden bzw. Einweg-Baugruppen ist ein schneller und einfacher Austausch der verschmutzten Teile möglich. Ebenso kann eine zusätzliche Vorrichtung zur Reinigung der Auffangvorrichtung bzw. der Elektroden vorhanden sein, so dass eine gleichmäßige und optimale Beseitigungswirkung durch die mindestens eine Elektrode sichergestellt werden kann.

Zur Reinigung der Anordnung kann erfindungsgemäß die Beseitigung der Ablagerungen von der Auffangvorrichtung durch eine mechanische Schwingungen erzeugende Vorrichtung vorgenommen werden. Somit kann der Reinigungsvorgang automatisch und kontinuierlich, z.B. ohne Reinigen von Teilen per Hand, durchgeführt werden. Die Abriebpartikel können z.B. vorteilhaft in einem Behälter gesammelt werden, in dem sie durch die mechanischen Schwingungen fallen. Dieser Behälter kann in größeren Zeitabständen entleert zu werden. Eine aufwendige und unhandliche Reinigung an der Auffangvorrichtung selbst bzw. der Elektrode oder einem Filter kann somit entfallen.

Eine günstige Anordnung ergibt sich, wenn die Vorrichtung zur elektrostatischem Entfernung von Schmutz- und Staubpartikeln möglichst in der Nähe einer diese verursachen schleift Kontaktanordnung angebracht ist.

Besonders vorteilhaft ist es, wenn mehrere der zuvor beschriebenen Ausführungsformen miteinander kombiniert werden, um eine besonders hohe Wirksamkeit des Systems zu erreichen.

Ein erfindungsgemäßes Verfahren zur Entfernung von Staub- und Schmutzpartikeln umfasst die Schritte Aufladen eines dünnen Drahtes mit Hochspannung und ionisieren der Luft. Weiterhin erfolgt ein Aufladen einer leitfähigen Oberfläche mit entgegengesetzter Polarität und führen einer Luftströmung über den dünnen Draht bis hin zur leitfähigen Oberfläche, sodass sich dort die Staub- und Schmutzpartikel ablagern.

Ein weiteres erfindungsgemäßes Verfahren zur Entfernung von Staub und Schmutzpartikeln insbesondere aus der Umgebung einer optischen Übertragungseinrichtung umfasst die Schritte Aufladen einer Rolle mit leitfähiger Oberfläche durch Anlegen einer hohen Spannung, drehen der Rolle der gleichzeitige Bewegung längs der zu reinigenden Oberfläche. Hierbei erfolgt die Bewegung in geringem Abstand zu Oberfläche. Mit der Bewegung der Rolle erfolgt gleichzeitig das Abstreifen der angezogenen Staub- und Schmutzpartikel von der Oberfläche der Rolle durch einen Abstreifer.

### Beschreibung der Zeichnungen

Die Erfindung wird nachstehend ohne Beschränkung des allgemeinen Erfindungsgedankens anhand von Ausführungsbeispielen unter Bezugnahme auf die Zeichnungen exemplarisch beschrieben.
Fig. 1 zeigt in allgemeiner Form schematisch eine erfindungsgemäße Vorrichtung.
Fig. 2 zeigt schematisch eine erfindungsgemäße Vorrichtung in der Draufsicht.
Fig. 3 zeigt eine Reinigungseinheit in Form einer Einrichtung zum Ausblasen von Staub und Schmutzpartikeln.
Fig. 4 zeigt eine Reinigungseinheit in Form einer Saugvorrichtung.
Fig. 5 zeigt eine Reinigungseinheit in Form einer Bürsteneinheit.
Fig. 6 zeigt eine erfindungsgemäße Vorrichtung mit einer Abdichtung durch Sperrluft.
Fig. 7 zeigt eine erfindungsgemäße Vorrichtung mit einer Labyrinthdichtung.
Fig. 8 zeigt eine erfindungsgemäße Vorrichtung mit einer Überdruckabdichtung.
Fig. 9 zeigt eine erfindungsgemäße Vorrichtung mit einer Bürstenabdichtung.
Fig. 10 zeigt eine erfindungsgemäße Vorrichtung mit einer Filzdichtung.
Fig. 11 zeigt eine erfindungsgemäße Vorrichtung mit einer Gleitringdichtung.
Fig. 12 zeigt eine erfindungsgemäße Vorrichtung mit Radialwellendichtungen.
Fig. 13 zeigt eine erfindungsgemäße Vorrichtung mit einer statischen Dichtung.
Fig. 14 zeigt eine erfindungsgemäße Vorrichtung mit einem mechanischen Filter.
Fig. 15 zeigt eine erfindungsgemäße Vorrichtung mit einem elektrostatischen Filter.
Fig. 16 zeigt eine Anordnung mit einer Membrandichtung.
Fig. 17 zeigt eine erfindungsgemäße Vorrichtung mit seitlich angeordnetem elektrostatischen Filter.
Fig. 18 zeigt eine erfindungsgemäße Vorrichtung zur Abstoßung geladener Staub- und Schmutzpartikel.
Fig. 19 zeigt eine erfindungsgemäße Vorrichtung mit einer Rolle zur Aufnahme geladener Staub- und Schmutzpartikel.
Fig. 20 zeigt eine erfindungsgemäße Vorrichtung mit einer kombinierten Micro-/Nano-Struktur beschichteten Oberfläche.

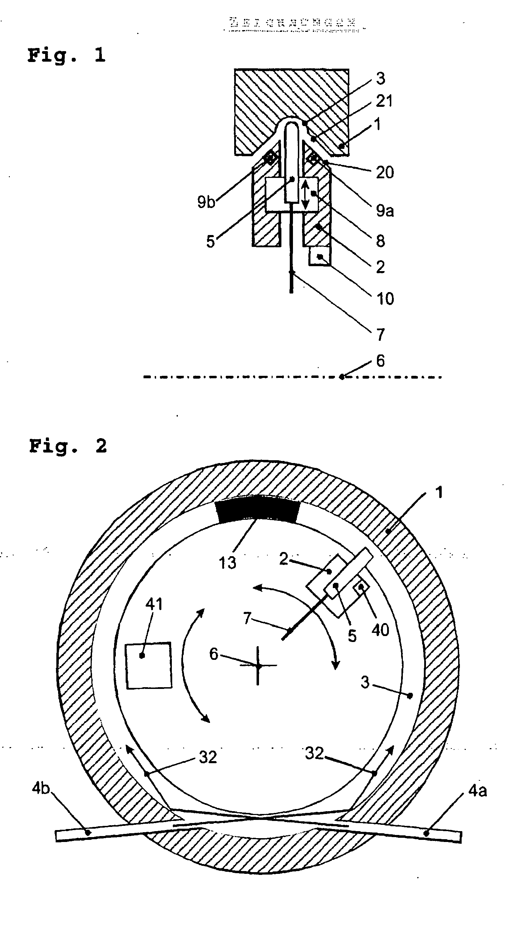

Fig. 1 zeigt in schematischer Form eine erfindungsgemäße Vorrichtung im Schnitt. Darin sind sowohl die erste Einheit 1 als auch die zweite Einheit 2 als Scheiben mit zentrischer Bohrung, welche um die Drehachse 6 drehbar gelagert sind, dargestellt. Der Lichtleiter 3 ist hier beispielhaft als auf der Innenseite verspiegelter Graben dargestellt. Er erstreckt sich um den gesamten Umfang der ersten Einheit. Im Eingriff mit diesem Graben ist ein zweiter Lichtkoppler 5, welcher an der zweiten Einheit 2 angeordnet ist. Dieser Lichtkoppler greift das in dem Lichtleiter geführte Licht ab und leitet es mit einer lichtleitenden Faser 7 weiter. Zur exakten Ausrichtung von Lichtleiter und zweitem Lichtkoppler in einer Achse ist eine Hydrodynamischen Lagerung sowie eine elektrodynamische Lageregelung vorgesehen. Die hydrodynamische Lagerung basiert auf einem dünnen Luftfilm, welche sich durch die Bewegung der beiden Einheiten gegeneinander zwischen der ersten Lagerfläche 21 und der zweiten Lagerfläche 20 ausgebildet wird. Zur Unterstützung sind beispielsweise zusätzliche Mittel zur Luftführung vorgesehen. Ein Aktuator 8 dient zur exakten Höheneinstellung des Lichtkopplers. Die Sensoren 9a und 9b dienen zur Ermittlung von Verschmutzungen.

Fig. 2 zeigt in schematischer Form eine erfindungsgemäße Vorrichtung in der Draufsicht. Eine erste Einheit 1 dient zur Aufnahme eines ringförmigen Lichtleiters 3. Dieser Lichtleiter ist beispielsweise ein auf der Innenseite verspiegelter Graben. Eine zweite Einheit 2 dreht sich gegenüber der ersten Einheit um die Drehachse 6. Die zweite Einheit enthält einen zweiten Lichtkoppler 5. Licht aus einem nicht dargestellten Sender wird bezogen auf das Modulationssignal gleichphasig mittels der beiden ersten Lichtkoppler 4a, 4b in den Lichtleiter 3 eingespeist. Das Licht vom ersten Lichtkoppler 4a läuft auf der rechten Seite der Abbildung bis zum Absorber 13. Gleichzeitig läuft das Licht des ersten Lichtkopplers 4b auf der linken Seite bis zum Absorber 13. Der Absorber ist symmetrisch in Bezug auf die Einkoppelstelle der ersten Lichtkoppler angeordnet, so dass die Lichtwege 32 auf beiden Seiten gleich lang sind. Der Abgriff des Lichts erfolgt mittels eines zweiten Lichtkopplers 5, welcher um die Drehachse 6 entlang der Bahn des Lichtleiters 3 drehbar gelagert ist und das abgegriffene Licht einem optischen Empfänger zuführt. Zur Vereinfachung ist der optische Empfänger ebenfalls nicht abgebildet. Eine Reinigungseinheit 40 ist in die zweite Einheit 2 integriert. Sie läuft mit dieser zweiten Einheit entlang dem Lichtleiter 3 und reinigt ihn entsprechend. Weiterhin ist eine unabhängige Reinigungseinheit 41 dargestellt, welche sich unabhängig von dem zweiten Lichtkoppler bewegen lässt.

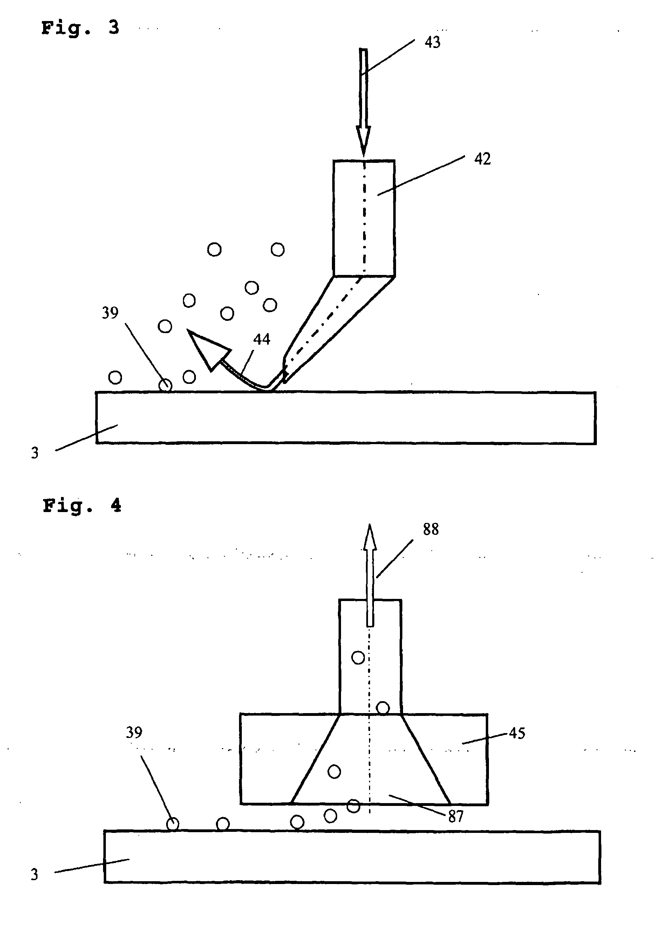

In Fig. 3 ist eine Reinigungseinheit in Form einer Einrichtung zum Ausblasen von Staub und Schmutzpartikeln dargestellt. Mittels einer Düse 42 wird ein Luftstrom 43 von einer Luftdruckquelle in Richtung des Lichtleiters Ziffer 3 geblasen. Der aus der Düse austretenden Luftstrom 44 wirbelt die Staubpartikel 39 auf und bläst diese vom Lichtleiter 3 fort.

Fig. 4 zeigt eine Reinigungseinheit in Form einer Saugvorrichtung. Ein Düsenblock 45, welcher in Form einer Düse ausgebildet ist, weist eine Absaugöffnung 87 auf, durch welche Luft von der Außenseite angesaugt wird. Der Luftstrom 88 reißt die Staubpartikel 39 mit und entfernt sie somit von der Oberfläche des Lichtleiters 3.

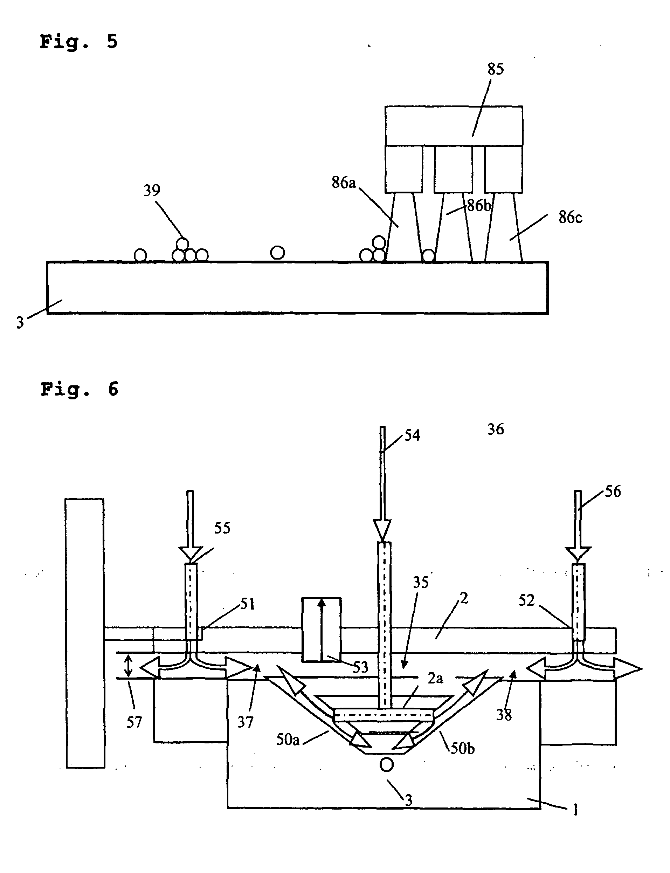

Fig. 5 zeigt eine Reinigungseinheit in Form einer Bürsteneinheit. Ein Bürstenträger 85 dient zur Aufnahme der Bürsten 86a, 86b, 86c. Die Bürsten werden entlang dem Lichtleiter 3 bewegt und kehren so auf der Oberfläche angelagerte Schmutzpartikel ab.

In Fig. 6 ist eine erfindungsgemäße Vorrichtung mit einer Abdichtung durch Sperrluft dargestellt. In dieser Darstellung ist zur Aufnahme des Lichtkopplers 5 ein Gleitkörper 2a, welcher mit der zweiten Einheit 2 verbunden ist vorgesehen. Dieser Gleitkörper 2a ist mit der zweiten Einheit 2 derart verbunden, dass er dieselbe Bewegung in Längsrichtung des Lichtleiters 3 ausführt, aber eine Stabilisierung in einer oder zwei Achsen senkrecht dazu bewirkt, so dass immer eine exakte Ausrichtung des Lichtkopplers 5 an der zweiten Einheit gegenüber dem Lichtleiter 3 gewährleistet ist. Eine besonders präzise und gleichzeitige Reibungsarme Lagerung dieses Gleitkörpers 2a gegenüber der ersten Einheit 1 lässt sich durch Luftlagerung erreichen. Eine solche Luftlagerung ist als besonders bevorzugtes Ausführungsbeispiel zur Erläuterung der erfindungsgemäßen Ausgestaltungen dargestellt. Selbstverständlich ist auch jede andere Art der Lagerung, wie beispielsweise mit Gleitlagern oder Wälzlagern möglich. Zur Luftlagerung sind hier beispielhaft zwei Düsen 50a, 50b dargestellt, aus welchen ein Zuluftstrom 54 von einer nicht dargestellten Druckluftquelle in Richtung der ersten Einheit 1 abgegeben wird, um einen Luftfilm zwischen dem Gleitkörper 2a und der ersten Einheit 1 aufzubauen. Die durch die Düsen eingebrachte Luft strömt seitlich zwischen dem Gleitkörper 2a und der ersten Einheit 1 ab. Sie kann den in der Zeichnung dargestellten Hohlraum zwischen den Teilen der ersten Einheit 1 und der zweiten Einheit 2 durch das Abluftventil 53 verlassen.

Zur Abdichtung mittels Sperrluft ist nun wenigstens eine Sperrluftdüse 51 vorgesehen, welche einen Sperrluftstrom 55 in den Zwischenraum zwischen erste Einheit 1 und zweite Einheit 2 derart abgibt, dass dieser aus dem Spalt zwischen den beiden Einheiten nach außen hin austritt und somit ein Eindringen von Staub und anderen Schmutzpartikeln in den Spalt zwischen den beiden Einheiten verhindert. Zur Abdichtung der zweiten Seite ist noch eine zweite Sperrluftdüse 52 gespeist durch einen Sperrluftstrom 56 eingezeichnet. Um ein optimales Abdichtungsergebnis zu erhalten, kann der Spalt 57 zwischen der ersten Einheit 1 und der zweiten Einheit 2 optimiert werden. Besonders günstig ist ein Spalt in einer Größenordnung von 0,03 Millimetern bis 0,1 Millimeter, wobei sich ein Überdruck gegenüber der Umgebung von 0,2 bar bis 0,5 bar als vorteilhaft erwiesen hat. Gleiches gilt für den Spalt 58. Für den Gegenstand der Erfindung ist die exakte Anzahl der Spalte unwesentlich, da dies meist eine Frage der Definition ist. So könnten beispielsweise auch die beiden Spalte 57 und 58 als ein einziger Spalt zwischen den beiden Einheiten betrachtet werden. Erfindungswesentlich ist, dass wenigstens ein Spalt zwischen den beiden Einheiten besteht.

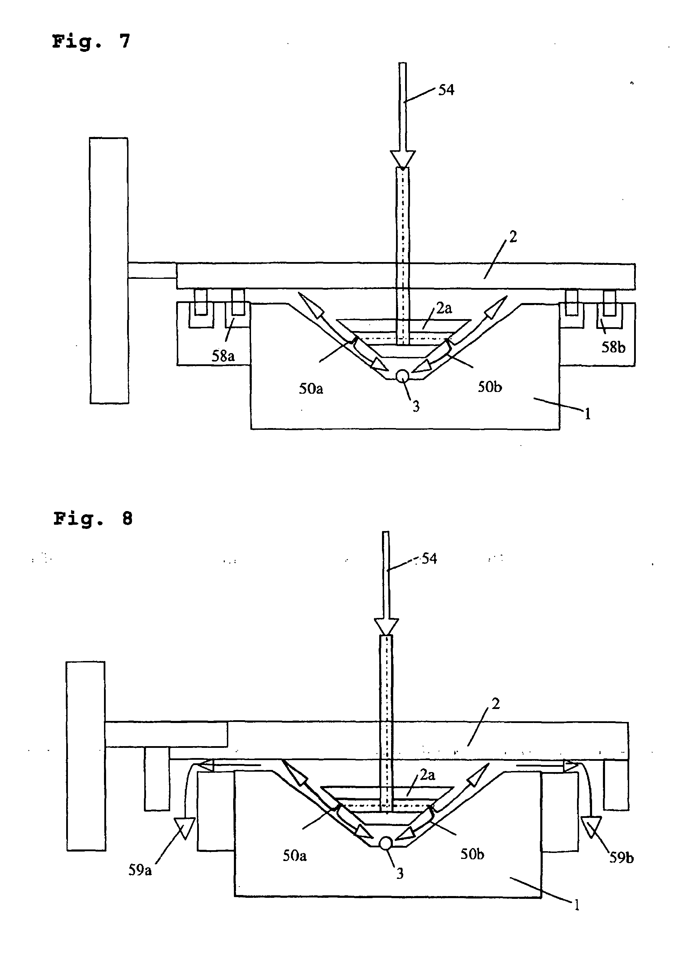

Fig. 7 zeigt eine erfindungsgemäße Vorrichtung mit einer Labyrinthdichtung. Hierbei wird das Eindringen von Staub und anderen Fremdkörpern in den Verschmutzung empfindlichen Bereich um den Lichtleiter 3 durch ein Labyrinth erschwert. Dieses Labyrinth wird vorzugsweise durch die Ausbildung einer ineinander greifenden Struktur aus Nuten beziehungsweise Stegen zwischen der ersten Einheit 1 und der zweiten Einheit 2 ausgebildet. In den hier dargestellten besonderen Ausführungsfall mit einer Luftlagerung tritt der in den Hohlraum eingeblasene Zuluftstrom 54 nach Austritt durch die Lagerung durch das Labyrinth nach außen und erschwert zusätzlich das Eindringen von Schmutz. Eine solche Labyrinthdichtung ist aber auch ohne den Luftstrom funktionsfähig.

Fig. 8 zeigt eine erfindungsgemäße Vorrichtung mit einer Überdruckabdichtung. Hierbei wird ähnlich, wie im vorhergehenden Beispiel, allerdings mit einer wesentlich größeren Luftmenge versucht, einen kontinuierlichen Luftstrom 59a beziehungsweise 59b nach außen hin aufrecht zu erhalten. Im Gegensatz zu Labyrinthdichtung ist die mechanische Ausführung hier wesentlich einfacher, allerdings ist ohne den Luftstrom nur eine sehr geringe Dichtwirkung zu erwarten. Dieses die vielen Anwendungsfällen aber nicht als besonders kritisch einzustufen, da im Ruhezustand einer Anlage auch der von außen aufgewirbelten Staub und damit das Verschmutzungsrisiko meist wesentlich geringer ist. Die Luftzufuhr kann wahlweise durch die Versorgung des Luftlagers oder durch zusätzliche Einströmöffnungen erfolgen. Vorzugsweise sind mehrere Einströmöffnungen auf dem Umfang der Anordnung verteilt vorgesehen.

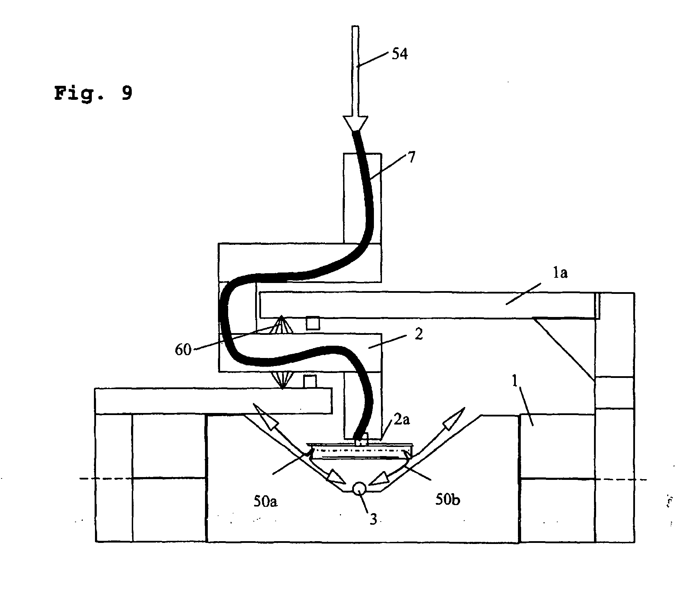

Fig. 9 zeigt eine erfindungsgemäße Vorrichtung mit einer Bürstenabdichtung. Hierbei sind Bürsten 60 vorgesehen, welche ein Eindringen von Staub und Schmutz in den Zwischenraum zwischen der ersten Einheit 1 und der zweiten Einheit 2 verhindern. Zur Verbesserung der Dichtwirkung können vorteilhafterweise zusätzliche Barrieren oder Labyrinthe vorgesehen sein. In diesem Ausführungsbeispiel ist fest an der ersten Einheit 1 eine Abdeckung 1a angebracht, so dass nur ein relativ schmaler Bereich für die zweite Einheit 2 zur Durchführung einer lichtleitenden Faser 7 sowie der hier beispielhaft benötigten zu Luft 54 realisiert ist.

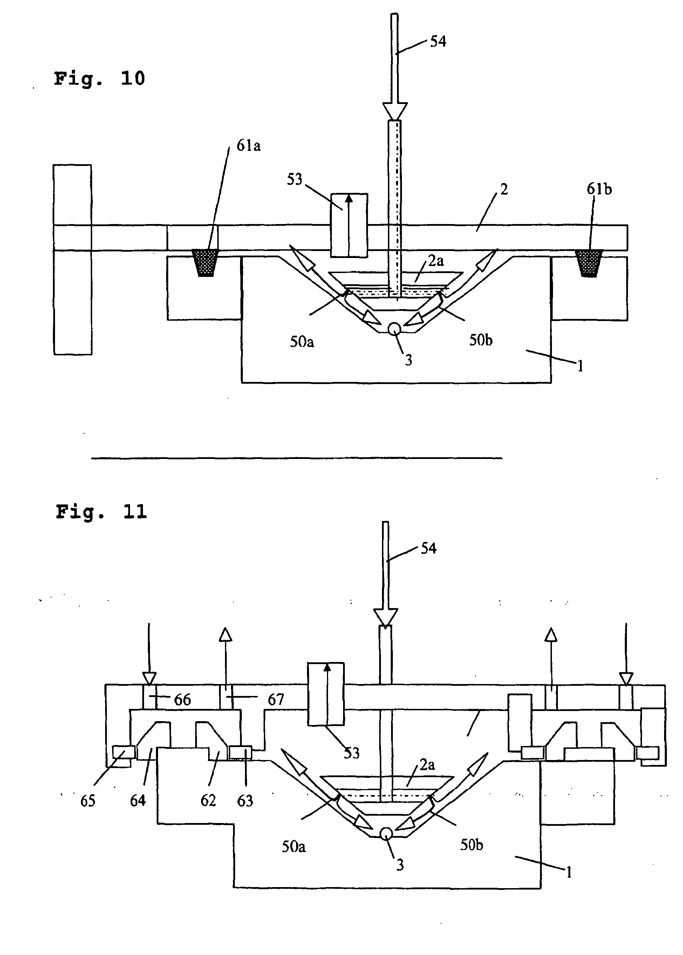

Fig. 10 zeigt eine erfindungsgemäße Vorrichtung mit einer Filzdichtung. Eine solche Filzdichtung kann ähnlich wie die zuvor beschriebene Bürstendichtung ausgeführt werden. Hier ist wieder beispielhaft die planare Anordnung von der ersten Einheit 1 und der zweiten Einheit 2 dargestellt. Zur Unterstützung der Filzdichtung kann auch hier der interne Überdruck herangezogen werden.

Fig. 11 zeigt eine erfindungsgemäße Vorrichtung mit einer Gleitringdichtung. Da die hier dargestellte Vorrichtung symmetrisch aufgebaut ist, wird nur auf die in der Abbildung links dargestellte Gleitringdichtung Bezug genommen. Grundsätzlich würde schon durch eine einfache Gleitringdichtung mit einem Gleitring 62 sowie dem Gegenring 63 eine Abdichtung des Innenraumes bewirken. Zur Verbesserung der Dichtwirkung ist noch ein zweiter Gleitring 64 mit dem entsprechenden Gegenring 65 vorgesehen. Weiterhin wird in den Zwischenraum, welcher durch die beiden Gleitringe und ihre Gegenringe abgeschlossen wird, durch einen Sperrlufteinlass 66 Sperrluft oder ein anderes Sperrmittel, wie beispielsweise eine Flüssigkeit eingebracht und durch den Sperrluftauslass 67 wieder abgeführt.

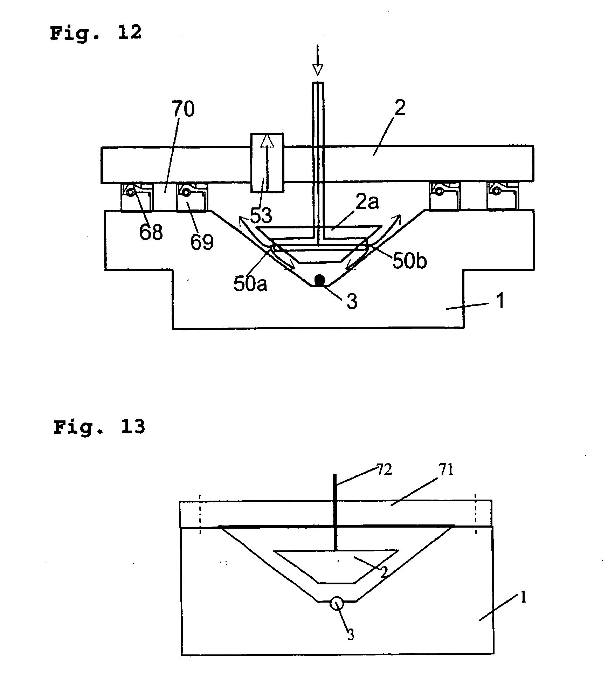

Fig. 12 zeigt eine erfindungsgemäße Vorrichtung mit Radialwellendichtungen. Hierbei sind Zur Abdichtung des Innenraumes Radialwellendichtringe 68,69 vorgesehen. In den Zwischenraum 70 zwischen den Radialwellendichtringen kann beispielsweise ein Schmiermittel eingebracht werden. Ebenso kann dieser, wie vor der dargestellt, mit Sperrluft beaufschlagt werden.

Fig. 13 zeigt eine erfindungsgemäße Vorrichtung mit einer statischen Dichtung. Hierbei wird im Gegensatz zu den zuvor beschriebenen Dichtsystemen der Raum über dem-Lichtleiter 3 vollständig statisch abgedichtet. Hierzu wird eine lichtdurchlässige Abdeckung 71, wie beispielsweise eine Glasscheibe oder Plexiglasscheibe über dem Lichtleiter 3 angebracht. Das Licht wird vorzugsweise in freier Strahlführung 72 durch die lichtdurchlässige Abdeckung 71 gekoppelt. Ein der zweiten Einheit 2 zugeordneter Gleitkörper 2a, welcher die zur Verkopplung mit dem Lichtleiter 3 notwendigen optischen Elemente enthält, kann beispielsweise durch einen magnetischen Mitnehmer mechanisch mit der zweiten Einheit 2 verkoppelt werden.

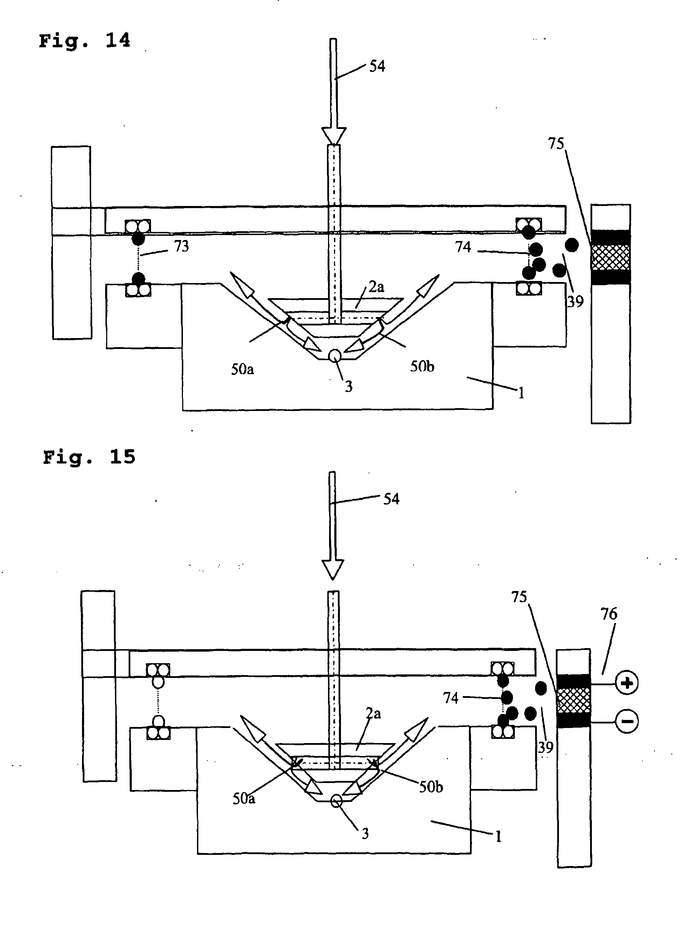

Fig. 14 zeigt eine erfindungsgemäße Vorrichtung mit einem mechanischen Filter. Hier ist ein mechanisches Filter 73 bzw. 74 vorgesehen, um das Eindringen von Staub- bzw. Schmutzpartikeln in den Zwischenraum zwischen der ersten Einheit 1 und der zweiten Einheit 2 zu verhindern. Als Filtermaterial kann beispielsweise eine Gaze eingesetzt werden. Als Ergänzung kann noch ein weiteres Filter 75 mit einer größeren Oberfläche, beispielsweise ein Faltenbalg-Filter eingesetzt werden. Damit kann ein schnelles Zusetzen des Filters verhindert bzw. verzögert werden. Eine zusätzliche Unterstützung der Filterwirkung bzw. eine Reinigung des Filters kann durch den von innen kommenden Zuluftstrom 54 erfolgen.

Fig. 15 zeigt eine erfindungsgemäße Vorrichtung mit einem elektrostatischen Filter. Ist grundsätzlich ähnlich wie die zuvor beschriebene Vorrichtung mit mechanischem Filter aufgebaut. Allerdings wird hier wahlweise an Stelle des mechanischen Filters kann 73, 74 bzw. der Gaze oder aber auch an Stelle des zusätzlichen Filters 75 eine elektrostatisches Filter eingesetzt. Die Versorgung dieses elektrostatischen Filters erfolgt über die Hochspannungs-Anschlüsse 76.

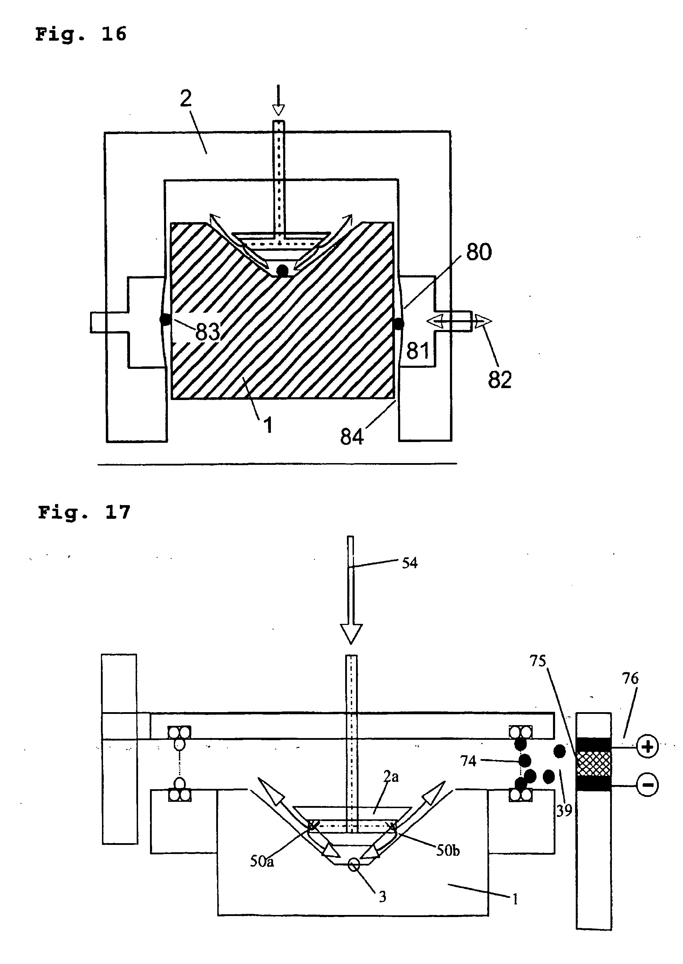

Fig. 16 zeigt eine Anordnung mit einer Membrandichtung Hierbei ist eine Dichtmembran 80 vorgesehen, welche vor einem Hohlraum 81 angeordnet ist. Durch Vakuum bzw. Druckluft 82 kann nun die Lage der Dichtmembran verändert werden. Im gezeigten Bild kann diese durch Beaufschlagung mit Druckluft an die Dichtung 83 des ersten Teils angedrückt und durch Beaufschlagung mit Vakuum abgehoben werden. Das Spaltmaß 84 gibt den Hub der Dichtmembran an. In der dargestellten Ausführungsform ist im Ruhezustand die entlastete Membran so dimensioniert, dass sie gerade an dem Dichtring anliegt. Damit ist auch im Ruhezustand eine gewisse minimale Abdichtung sichergestellt. Zur Erhöhung der Dichtwirkung kann nun der Hohlraum bzw. die Rückseite der Membran mit Überdruck beaufschlagt werden. Im Betrieb wird durch Vakuum die Luft aus dem Hohlraum entzogen, so dass sich die Dichtmembran von der Dichtung abhebt und ein Betrieb bei minimaler Reibung möglich ist.

Fig. 17 zeigt eine erfindungsgemäße Vorrichtung mit einem seitlich angebrachten elektrostatischen Filter. Durch den Zuluftstrom 54, welcher durch die Düsen 50a und 50b in dem Gleitkörper 2a der zweiten Einheit 2 ausströmt und den für die Luftlagerung notwendigen Luftfilm bildet, wird eine Strömung aus dem Lagerungs-Bereich nach außen erzeugt. Die durch diese Strömung mitgerissenen Staub und Schmutzpartikel 39 werden von einem elektrostatischen Filter 75 ausgefiltert. Der Filter 74 ist ein zusätzlicher Filter aus Gaze, um den Innenbereich der Lagerung gegen Einbringen von Staub von Schmutz zu schützen. Der elektrostatische Filter 75 wird über die Spannungsanschlüsse 76 gespeist. Zur Speisung kann ein Hochspannungsnetzteil oder eine Vorrichtung zur Erzeugung der hohen Spannung durch elektrostatisches Aufladung, z.B. auf Grund von Reibungselektrizität durch die Bewegung der ersten und zweiten Einheit gegeneinander vorgesehen werden.

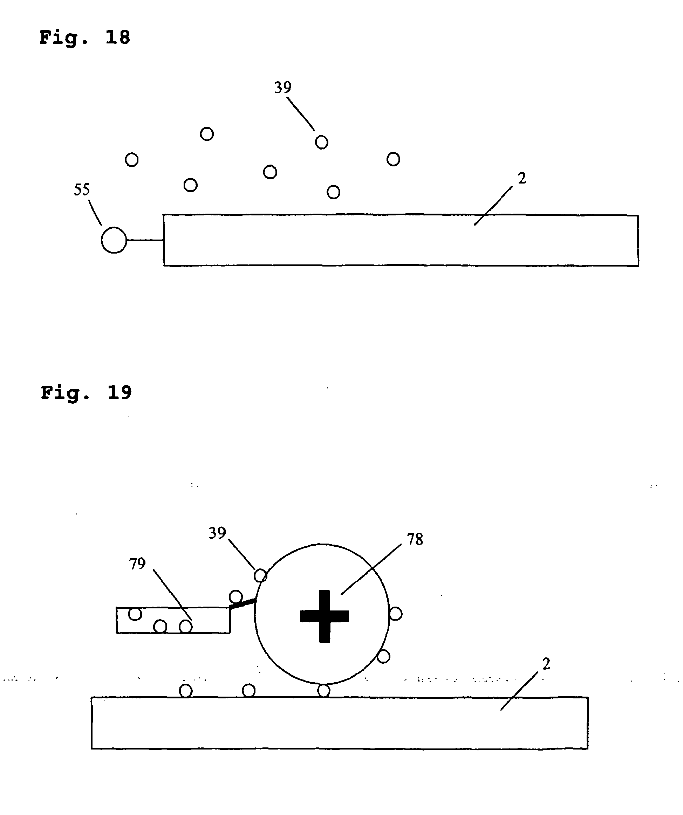

Fig. 18 zeigt eine andere Art der elektrostatischen Reinhaltung der Oberfläche insbesondere der zweiten Einheit 2. Hierbei wird diese durch eine statische Spannungsversorgung 90 mit Hochspannung beaufschlagt, so dass geladene Staub- und Schmutzpartikel 39 von dieser abgestoßen werden. Es kann eine zusätzliche Fläche mit umgekehrter Polarität vorgesehen werden, welche nun diese Partikel anzieht und auch festhält. Hierzu kann die Oberfläche eine Beschichtung, vorzugsweise aus einem Polymer mit einer hohen Adhäsion aufweisen.

Fig. 19 zeigt eine erfindungsgemäße Vorrichtung mit einer Rolle zur Aufnahme geladener Staub- und Schmutzpartikel. Hierbei wird eine Rolle 78, welche beispielsweise durch eine Spannungsversorgung aufgeladen wird, in der Nähe der zu reinigenden Oberfläche, bevorzugt der zweiten Einheit bewegt. Diese nimmt nun geladene Staub- und Schmutzpartikel auf. Damit immer eine unverschmutzte Oberfläche der Rolle zur Verfügung steht, wird diese ebenfalls mit einer Drehbewegung weiterbewegt. Im vorliegenden Beispiel ist die Drehrichtung entgegen dem Uhrzeigersinn. Weiterhin ist ein Abscheider 79 vorgesehen, welcher die auf der Rolle befindlichen Staub- und Schmutzpartikel aufnimmt. Die Oberfläche der Rolle kann unterschiedlich gestaltet sein. So kann die Rolle eine durchgängig leitende Oberfläche aufweisen, wobei sie in ihrer Gesamtheit von einer Hochspannungsversorgung gespeist wird. Ebenso kann sie aber auch ähnlich wie die bekannten Fotoleitertrommeln in Fotokopierern oder Laserdruckern ausgebildet sein, wobei die Aufladung der Oberfläche von einer in der Nähe der Oberfläche angebrachten Coronaelektrode aus erfolgt.

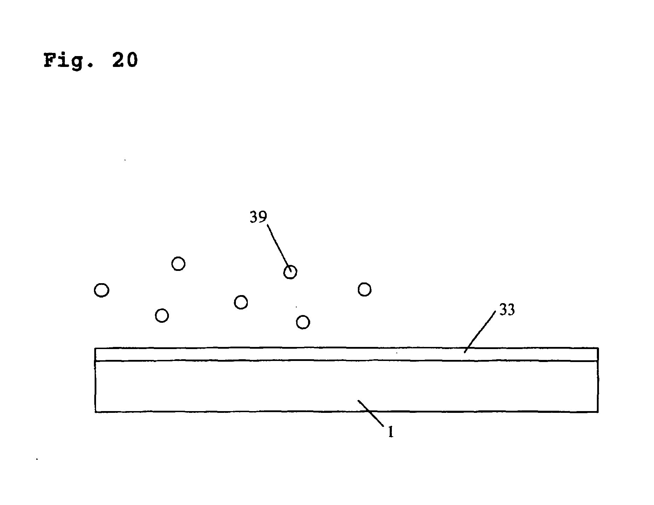

In Figur 20 ist noch eine kombinierte Micro-/NanoBeschichtung 33 dargestellt, welche durch einen Effekt, wie er durch die Lotus-Blüte bekannt ist, ein Ansetzen von Verunreinigungen 39 auf der Oberfläche verhindert. Eine solche Struktur kann wahlweise an der ersten Einheit 1, der zweiten Einheit 2 oder an anderen Teilen, wie dem Lichtleiter 3 angebracht sein. Hierzu ist vorzugsweise eine Mikrostruktur mit Erhebungen im Mikrometer-Bereich kombiniert mit einer darüber liegenden Nano-Struktur mit Erhebungen im Nanometer-Bereich Eine derart beschichtete Oberfläche kann nun so ausgeführt werden, dass beispielsweise Verunreinigungen vollständig von den kritischen Bereichen der Oberfläche (optische Komponenten) weggeblasen werden können. Ebenso könnten diese gezielt in einen Auffangbehälter oder in ein Auffangfilter geleitet werden.

Selbstverständlich können die hier beschriebenen verschiedenen Ausgestaltungen der Erfindung miteinander kombiniert werden, um eine bessere Filterung von Schmutz- und Staubpartikeln zu erreichen.

### Bezugszeichenliste

- 1: Erste Einheit
- 1a: Abdeckung
- 2: Zweite Einheit
- 3: Lichtleiter
- 4: Erster Lichtkoppler an der ersten Einheit
- 5: Lichtkoppler an der zweiten Einheit
- 6: Drehachse der Drehung zwischen erster und zweiter Einheit
- 7: Lichtleitende Faser
- 8: Aktuator
- 9: Sensor
- 10: Steuereinheit
- 11: Referenzspur
- 13: Absorber
- 20: zweite Lagerfläche
- 21: erste Lagerfläche
- 32: Lichtstrahl
- 33: Mikro / Nanobeschichtung
- 35: Innenbereich
- 36: Außenbereich
- 37: erster Spalt
- 38: zweiter Spalt
- 39: Staubpartikel bzw. Schmutzschicht
- 40: in die zweite Einheit integrierte. Reinigungseinheit
- 41: unabhängige Reinigungseinheit
- 42: Düse
- 43: Luftstrom von.einer Luftdruckquelle
- 44: Luftstrom aus der Düse auftretend

- 45: Düsenblock
- 46: Staubpartikel bzw. Schmutzschicht
- 50: Düse
- 51: erste Sperrluftdüse
- 52: zweite Sperrluftdüse
- 53: Abluftventil
- 54: Zuluftstrom
- 55: erster Sperrluftstrom
- 56: zweiter Sperrluftstrom
- 57: Spalthöhe zwischen erster und zweiter Einheit
- 58: Labyrinth
- 59: Luftstrom
- 60: Bürsten
- 61: Filzring
- 62: Gleitring
- 63: Gegenring
- 64: Gleitring
- 65: Gegenring
- 66: Sperrlufteinlass
- 67: Sperrluftauslass
- 68: Radialwellendichtring
- 69: Radialwellendichtring
- 70: Kammer für Schmiermittel
- 71: Abdeckung - lichtdurchlässig
- 72: Lichtstrahl
- 73: Filter (Gaze)
- 74: Filter (Gaze)
- 75: Filter
- 76: Hochspannungs-Anschlüsse
- 78: Rolle
- 79: Abscheider
- 80: Dichtmembran
- 81: Hohlraum
- 82: Vakuum
- 83: Dichtung
- 84: Spaltmaß
- 85: Bürstenträger
- 86: Bürsten
- 87: Absaugöffnung
- 88: Luftstrom mit Schmutzpartikeln
- 90: Statische Spannungsversorgung

## Patentansprüche

1. Vorrichtung zum Einsatz in Computertomographen mit freiem Innenbereich zur Übertragung modulierter optischer Signale zwischen einer ersten Einheit (1) und einer zweiten Einheit (2), wobei die erste Einheit gegenüber der zweiten Einheit drehbar gelagert ist, umfassend
- einen Lichtleiter (3) entlang einer Kreisbahn an der ersten Einheit,
- wenigstens einen mit dem Lichtleiter verbundenen ersten Lichtkoppler (4) zur Lichtein- bzw. Auskopplung in den Lichtleiter,
- wenigstens einen zweiten Lichtkoppler (5), welcher an der zweiten Einheit angeordnet ist, und gegenüber dem Lichtleiter beweglich ist, zur Lichtein- bzw. Auskopplung in den Lichtleiter,
- wenigstens eine Reinigungseinheit (40) zur Entfernung von Schmutz und/oder Staubpartikeln vorgesehen ist, welche mit wenigstens einer zweiten Einheit (2) derart verbunden ist, so dass diese zusammen mit der zweiten Einheit entlang des Lichtleiters bewegbar ist, **dadurch gekennzeichnet, dass** eine Einrichtung zum Ausblasen von Staub und Schmutzpartikeln mit einer Düse (42) zur Erzeugung eines Luftstroms (44), welcher zumindest auf den Lichtleiter (3) gerichtet ist, umfasst.

2. Vorrichtung nach Anspruch 1,
**dadurch gekennzeichnet, dass**
wenigstens eine Reinigungseinheit (41) vorgesehen ist, welche unabhängig von einem Lichtkoppler (5) entlang des Lichtleiters bewegbar ist.

3. Vorrichtung nach Anspruch 1,
**dadurch gekennzeichnet, dass**
wenigstens ein Sensor zur Erkennung von Verschmutzungen auf der zu reinigenden Oberfläche vorgesehen ist, und dieser einer optionalen Steuereinheit die Verschmutzung signalisiert, so dass diese abhängig von Verschmutzungsgrad die Einwirkzeit und/oder Reinigungsintensität der Reinigungseinheit einstellt.

4. Vorrichtung nach Anspruch 1,
**dadurch gekennzeichnet, dass**
wenigstens eine Reinigungseinheit (40, 41) eine Saugvorrichtung mit einer Absaugöffnung durch welche die Staubpartikel und Schmutzpartikel von der Oberfläche des Lichtleiters (3) abgesaugt werden, umfasst.

5. Vorrichtung nach Anspruch 1,
**dadurch gekennzeichnet, dass**
wenigstens eine Reinigungseinheit (40, 41) eine Bürsteneinheit aus einem Bürstenträger (52) mit wenigstens einer Bürste (53) umfasst.

6. Verfahren zur Reinigung der Oberfläche eines Lichtleiters (3) in einer Vorrichtung nach Anspruch 1,
**gekennzeichnet durch**
Erzeugen eines Luftstroms (44) mit einer Düse (42) und Ausblasen von Staub und Schmutzpartikeln von der Oberfläche des Lichtleiters (3).

7. Verfahren zur Reinigung der Oberfläche eines Lichtleiters (3) in einer Vorrichtung nach Anspruch 1,
**gekennzeichnet durch**
Absaugen von Staubpartikeln und Schmutzpartikeln von der Oberfläche des Lichtleiters (3).

8. Verfahren zur Reinigung der Oberfläche eines Lichtleiters (3) in einer Vorrichtung nach Anspruch 1,
**gekennzeichnet durch**
Abbürsten von Staubpartikeln und Schmutzpartikeln von der Oberfläche des Lichtleiters (3).

9. Verfahren zur Reinigung der Oberfläche eines Lichtleiters (3) in einer Vorrichtung nach Anspruch 1,
**gekennzeichnet durch**
Abgabe eines Dampfstromes auf den Lichtleiter (3).

10. Verfahren zur Reinigung der Oberfläche eines Lichtleiters (3) in einer Vorrichtung nach Anspruch 1,
**gekennzeichnet durch**
Abgabe einer Reinigungsflüssigkeit auf den Lichtleiter (3).

11. Vorrichtung nach Anspruch 1,
**dadurch gekennzeichnet, dass**
eine Vorrichtung zur elektrostatischen Entfernung von Schmutz- und Staubpartikeln vorgesehen ist.

12. Vorrichtung nach Anspruch 11,
**dadurch gekennzeichnet, dass**
wenigstens ein elektrostatisches Luftfilter vorgesehen ist, welches wenigstens eine mit Hochspannung beaufschlagbare Elektrode aufweist, welche geladene Staub- und Schmutzpartikel anzieht.

13. Vorrichtung nach Anspruch 11,
**dadurch gekennzeichnet, dass**
wenigstens eine Coronaelektrode, vorzugsweise als dünner Draht, welcher mit Hochspannung beaufschlagbar ist, zur elektrostatisches Aufladung der in der der die Anordnung umgebenden Luft enthaltenen Staub- und Schmutzpartikel vorgesehen ist.

14. Vorrichtung nach Anspruch 11,
**dadurch gekennzeichnet, dass**
wenigstens ein Mittel zur Erzeugung einer auf das elektrostatische Luftfilter und/oder die Coronaelektrode gerichteten Luftströmung, insbesondere ein Gebläse oder Ventilator vorgesehen ist.

15. Vorrichtung nach Anspruch 11,
**dadurch gekennzeichnet, dass**
wenigstens ein Mittel zur Luftführung, wie Luftleitbleche oder Luftkanäle vorgesehen ist.

16. Vorrichtung nach Anspruch 11,
**dadurch gekennzeichnet, dass**
ein weiteres Luftfilter, beispielsweise ein Maschenfilter oder Papierfilter, zusätzlich vorgesehen ist.

17. Vorrichtung nach Anspruch 11,
**dadurch gekennzeichnet, dass**
wenigstens ein Mittel zur Aufnahme von Staub- und Schmutzpartikeln nahe über der Oberfläche einer der beiden Einheiten angeordnet ist und mit Hochspannung beaufschlagbar ist, so dass geladene Staub- und Schmutzpartikel von diesem angezogen werden.

18. Vorrichtung nach Anspruch 17,
**dadurch gekennzeichnet, dass**
ein Mittel zur Aufnahme von Staub- und Schmutzpartikeln als drehbarer Körper, vorzugsweise in rotationssymmetrischer Form ausgebildet ist.

19. Vorrichtung nach Anspruch 11,
**dadurch gekennzeichnet, dass**
wenigstens eine Elektrode eine Oberfläche mit hoher Adhäsion für Staub- und Schmutzpartikel ausgebildet ist.

20. Vorrichtung nach Anspruch 11,
**dadurch gekennzeichnet, dass**
wenigstens eine Elektrode als austauschbare Einweg-Elektrode ausgebildet ist.

21. Vorrichtung nach Anspruch 11,
**dadurch gekennzeichnet, dass** eine Filteranordnung vorgesehen und zumindest ein Teil der Filteranordnung als austauschbare Einweg-Baugruppe ausgebildet ist.

22. Vorrichtung nach Anspruch 11,
**dadurch gekennzeichnet, dass**
im Falle des Vorhandenseins einer mechanischen Schleifkontaktanordnung wenigstens eine Vorrichtung zur elektrostatischen Entfernung von Schmutz- und Staubpartikeln räumlich der mechanischen Schleifkontaktanordnung zugeordnet ist, um die Staub- und Schmutzpartikel unmittelbar an der Stelle ihres Entstehens aus der Luft zu filtern.

23. Vorrichtung nach Anspruch 11,
**dadurch gekennzeichnet, dass**
eine Vorrichtung zur Erzeugung mechanischer Schwingungen oder Stöße vorgesehen ist, welche die Vorrichtung zur Filterung von Ablagerungen befreit.

## Claims

1. Device for use in computer tomographs with a free inner region for transmitting modulated optical signals between a first unit (1) and a second unit (2), the first unit being rotatably mounted with respect to the second unit, comprising:
- an optical wave guide (3) along a circular path on the first unit,
- at least one first optocoupler (4) connected to the optical wave guide (3) for coupling light into or out of the optical wave guide (3),
- at least one second optocoupler (5), which is arranged on the second unit (2) and can be moved with respect to the optical wave guide (3), for coupling light into or out of the optical wave guide (3),
- at least one cleaning unit (4) for removing dirt and/or particles of dust which is connected to at least one second unit (2) in such a way that it can be moved together with the second unit (2) along the optical wave guide (3), **characterised by** a device for blowing out particles of dust and dirt comprising a nozzle (42) for generating an air flow (44) which is directed at least onto the optical wave guide (3).

2. Device according to claim 1, **characterised in that** at least one cleaning unit (41) is provided which can be moved along the optical wave guide independently of an optocoupler (5).

3. Device according to claim 1, **characterised in that** at least one sensor is provided for detecting soiling on the surface to be cleaned and **in that** the sensor signals the soiling to an optional control unit, so it adjusts the application time and/or cleaning intensity of the cleaning unit as a function of the degree of soiling.

4. Device according to claim 1, **characterised in that** at least one cleaning unit (40, 41) comprises a suction device with a suction aperture through which the particles of dust and particles of dirt are removed by suction from the surface of the optical wave guide (3).

5. Device according to claim 1, **characterised in that** at least one cleaning unit (40, 41) comprises a brush unit comprising a brush carrier (52) with at least one brush (53).

6. Method for cleaning the surface of an optical wave guide (3) in a device according to claim 1, **characterised by** generating an air flow (44) with a nozzle (42) and blowing out particles of dust and dirt from the surface of the optical wave guide (3).

7. Method for cleaning the surface of an optical wave guide (3) in a device according to claim 1, **characterised by** suction removal of particles of dust and particles of dirt from the surface of the optical wave guide (3).

8. Method for cleaning the surface of an optical wave guide (3) in a device according to claim 1, **characterised by** brushing away particles of dust and particles of dirt from the surface of the optical wave guide (3).

9. Method for cleaning the surface of an optical wave guide (3) in a device according to claim 1, **characterised by** emission of a flow of steam onto the optical wave guide (3).

10. Method for cleaning the surface of an optical wave guide (3) in a device according to claim 1, **characterised by** emission of a cleaning liquid onto the optical wave guide (3).

11. Device according to claim 1, **characterised in that** a device is provided for electrostatic removal of particles of dirt and dust.

12. Device according to claim 11, **characterised in that** at least one electrostatic air filter is provided which comprises at least one electrode that can be subjected to high voltage and which attracts charged particles of dust and dirt.

13. Device according to claim 11, **characterised in that** at least one corona electrode, preferably as a thin wire which can be subjected to high voltage, is provided for electrostatically charging the particles of dust and dirt contained in the air surrounding the arrangement.

14. Device according to claim 11, **characterised in that** at least one means, in particular a fan or ventilator, is provided for generating a flow of air directed onto the electrostatic air filter and/or the corona electrode.

15. Device according to claim 11, **characterised in that** at least one means for conducting air, such as air baffles or air ducts, is provided.

16. Device according to claim 11, **characterised in that** a further air filter, for example a mesh filter or paper filter, is also provided.

17. Device according to claim 11, **characterised in that** at least one means for receiving particles of dust and dirt is arranged close to and above the surface of one of the two units and can be subjected to high voltage, to attract charged particles of dust and dirt thereby.

18. Device according to claim 17, **characterised in that** a means for receiving particles of dust and dirt is constructed as a rotatable body, preferably in rotationally symmetrical form.

19. Device according to claim 11, **characterised in that** at least one electrode is constructed as a surface with high adhesion for particles of dirt and dust.

20. Device according to claim 11, **characterised in that** at least one electrode is replaceable and disposable electrode.

21. Device according to claim 11, **characterised in that** a filter arrangement is provided and at least some of the filter arrangement is constructed as a replaceable, disposable assembly.

22. Device according to claim 11, **characterised in that** where a mechanical sliding contact arrangement exists at least one device for electrostatically removing particles of dirt and dust is sterically associated with the mechanical sliding contact arrangement to filter the particles of dust and dirt from the air directly at their point of origin.

23. Device according to claim 11, **characterized in that**, it comprises a device for exciting mechanical oszillations or impacts which free the device for removal from disposals.

## Revendications

1. Dispositif destiné à être utilisé dans des tomodensitomètres avec un espace intérieur libre afin de transférer des signaux optiques modulés entre une première unité (1) et une seconde unité (2), la première unité étant logée en face de la seconde unité de manière à pouvoir pivoter, comprenant
- un conducteur optique (3) le long d'une trajectoire circulaire sur la première unité,
- au moins un premier coupleur optoélectronique (4) relié au conducteur optique destiné au couplage /découplage par lumière dans le conducteur optique,
- au moins un second coupleur optoélectronique (5), qui est disposé sur la seconde unité et qui peut se déplacer vis-à-vis du conducteur optique, destiné au couplage/découplage par lumière dans le conducteur optique,
- au moins une unité de nettoyage (40) pour enlever des impuretés et/ou des particules de poussière, qui est relié à au moins une seconde unité (2) de manière à être mobile avec cette dernière le long du conducteur optique, **caractérisé en ce qu'**il comprend un dispositif pour purger la poussière et les particules d'impuretés, avec une tuyère (42) dirigée au moins vers le conducteur optique (3) pour générer un flux d'air (44).

2. Dispositif selon la revendication 1,
**caractérisé en ce qu'**est prévue au moins une unité de nettoyage (41) déplaçable le long du conducteur optique indépendamment d'un coupleur optoélectronique (5).

3. Dispositif selon la revendication 1,
**caractérisé en ce qu'**est prévu au moins un capteur de détection d'impuretés sur la surface à nettoyer et signale à cette unité de commande optionnelle l'impureté, de sorte qu'elle règle le temps d'action et/ou l'intensité de nettoyage de l'unité de nettoyage en fonction du degré d'encrassement.

4. Dispositif selon la revendication 1,
**caractérisé en ce qu'**au moins une unité de nettoyage (40, 41) comprend un dispositif d'aspiration avec un orifice d'aspiration à travers lequel les particules de poussière et les particules d'impuretés sont aspirées de la surface du conducteur optique (3.).

5. Dispositif selon la revendication 1,
**caractérisé en ce qu'**au moins une unité de nettoyage (40, 41) comprend une unité à brosse composée d'un support de brosse (52) avec au moins une brosse (53).

6. Procédé pour le nettoyage de la surface d'un conducteur optique (3) dans un dispositif selon la revendication 1,
**caractérisé par** la génération d'un flux d'air (44) avec une tuyère (42) et la purge de poussière et de particules d'impuretés de la surface du conducteur optique (3).

7. Procédé pour le nettoyage de la surface d'un conducteur optique (3) dans un dispositif selon la revendication 1,
**caractérisé par** l'aspiration de particules de poussière et de particules d'impuretés de la surface du conducteur optique (3).

8. Procédé pour le nettoyage de la surface d'un conducteur optique (3) dans un dispositif selon la revendication 1,
**caractérisé par** l'élimination par brossage de particules de poussière et de particules d'impuretés de la surface du conducteur optique (3).

9. Procédé pour le nettoyage de la surface d'un conducteur optique (3) dans un dispositif selon la revendication 1,
**caractérisé par** l'émission d'un flux de vapeur sur le conducteur optique (3).

10. Procédé pour le nettoyage de la surface d'un conducteur optique (3) dans un dispositif selon la revendication 1,
**caractérisé par** l'émission d'un liquide de nettoyage sur le conducteur optique (3).

11. Dispositif selon la revendication 1,
**caractérisé en ce qu'**est prévu un dispositif d'élimination électrostatique de particules d'impuretés et de poussière.

12. Dispositif selon la revendication 11,
**caractérisé en ce qu'**est prévu au moins un filtre à air électrostatique qui comporte au moins une électrode pouvant être mis sous haute tension qui attire des particules de poussière et d'impuretés chargées.

13. Dispositif selon la revendication 11,
**caractérisé en ce qu'**est prévue au moins une électrode corona, de préférence sous forme de fil mince pouvant être mis sous haute tension, pour la charge électrostatique des particules de poussière et d'impuretés contenues dans l'air ambiant du dispositif.

14. Dispositif selon la revendication 11,
**caractérisé en ce**
**qu'**est prévu au moins un moyen de génération d'un flux d'air dirigé vers le filtre à air électrostatique et/ou l'électrode corona, en particulier une soufflante ou un ventilateur.

15. Dispositif selon la revendication 11,
**caractérisé en ce qu'**est prévu au moins un moyen pour le guidage d'air, comme des déflecteurs d'air ou des canaux à air.

16. Dispositif selon la revendication 11,
**caractérisé en ce qu'**est prévu un filtre à air supplémentaire, par exemple un filtre en mailles ou un filtre en papier, est prévu.

17. Dispositif selon la revendication 11,
**caractérisé en ce qu'**au moins un moyen de récupération de particules de poussière et d'impuretés est disposé juste au-dessus de la surface d'une des deux unités et peut être mis sous haute tension, de manière à ce que des particules de poussière et d'impuretés soient attirées par celui-ci.

18. Dispositif selon la revendication 17,
**caractérisé en ce qu'**un moyen de récupération de particules de poussière et d'impuretés est réalisé sous forme de corps rotatif, de préférence dans une forme à symétrie de révolution.

19. Dispositif selon la revendication 11,
**caractérisé en ce qu'**au moins une électrode est dotée d'une surface à forte adhérence pour des particules de poussière et d'impuretés.

20. Dispositif selon la revendication 11,
**caractérisé en ce qu'**au moins une électrode est réalisée sous forme d'électrode jetable interchangeable.

21. Dispositif selon la revendication 11,
**caractérisé en ce qu'**est prévu un dispositif de filtres et **en ce qu'**au moins une partie du dispositif de filtres est réalisée sous forme de sous-groupe jetable interchangeable.

22. Dispositif selon la revendication 11,
**caractérisé en ce qu'**en présence d'un dispositif mécanique à contact à frottement, au moins un dispositif d'élimination électrostatique de particules d'impuretés et de poussière est affecté dans l'espace au dispositif mécanique à contact par frottement, afin d'enlever les particules de poussière et d'impuretés de l'air par filtrage directement là où elles sont générées.

23. Dispositif selon la revendication 11,
**caractérisé en ce qu'**est prévu un dispositif pour la génération de vibrations ou de chocs mécaniques qui libère le dispositif de filtrage de dépôts.
